# EUROPEAN PATENT SPECIFICATION

(11) **EP 1 834 959 B1**
(45) Date of publication and mention of the grant of the patent: **08.08.2012**
(21) Application number: 07106196.4
(22) Date of filing: 05.11.2001
(51) Int. Cl.: C07J 1/00, A61P 39/00, A61K 31/565

(54) **Tert-butyl-substituted aromatic steroids having cytoprotective activity**
Tert-butyl-substituierte aromatische Steroide mit zytoprotektiver Wirkung
Stéroides aromatiques substituées par tert-butyl comportant une activité cytoprotectrice

(30) Priority: 03.11.2000 US 245791 P
(43) Date of publication of application: 19.09.2007
(62) Divisional of application: 01992033.9
(73) Proprietor: Washington University, St. Louis, MO 63130 (US)
(72) Inventor: Covey, Douglas F., St.Louis, MO Missouri 63130 (US)
(74) Representative: Smaggasgale, Gillian Helen

(56) References cited:
- US-A- 5 843 934
- LUNN ET AL.: "the adamantyl carbonium ion as a dehydrogenating agent, its reactions with estrone" TETRAHEDRON, vol. 24, no. 23, 1968, pages 6773-6776, XP002446726
- MILLER ET AL.: "In vitro antioxidant effects of estrogens with ahindered 3-OH function on the copper-induced oxidation of low-density lipoprotein" STEROIDS, vol. 61, 1996, pages 305-308, XP002446727

## Description

### BACKGROUND OF THE INVENTION

The present disclosure generally relates to modified, hydroxy-substituted or hydroxy-bearing aromatic structures having cytoprotective activity, as well as to a treatment process involving the administration of an effective dosage thereof. More specifically, the present invention is directed to phenolic compounds or catecholic compounds which have been modified by the attachment of a tertiary-butyl substituent. Such compounds have been found to possess enhanced cytoprotective activity, as compared to their respective analogs which do not contain such a substituent. This activity may be conferred to a population of cells in a subject upon the administration of an effective dosage of the modified compound.

Cytodegenerative diseases are characterized by the dysfunction and death of cells, this dysfunction or death in the case of neurons leading to the loss of neurologic functions mediated by the brain, spinal cord and the peripheral nervous system. Examples of chronic neurodegenerative diseases include Alzheimer's disease, peripheral neuropathy (secondary to diabetes or chemotherapy treatment), multiple sclerosis, amyotrophic lateral sclerosis, Huntington's disease and Parkinson's disease, Creutzfeldt-Jakob disease and AIDs dementia. Normal brain aging is also associated with loss of normal neuronal function and may entail the depletion of certain neurons. Examples of acute neurodegenerative disease are stroke and multiple infarct dementia. Sudden loss of neurons may also characterize the brains of patients with epilepsy and those that suffer hypoglycemic insults and traumatic injury of the brain, peripheral nerves or spinal cord.

There continues to be a need for treatments that protect cells from cell death resulting from episodes of, for example, disease, trauma, isolation and removal of tissues or cells from the body, or exposure to toxins. This need extends to, among other things: (i) treatments for nerve cell loss associated with chronic or acute neurodegenerative disorders or trauma; (ii) treatments to minimize tissue damage resulting from ischemia where ischemia may occur as a result of stroke, heart disease, a transplantation event, or other event resulting in a cut-off in nutritional supply to tissues; and, (iii) treatments to modulate cell death associated with other degenerative conditions (such as osteoporosis or anemia). The absence of an effective cytoprotective therapy can result in either loss of life or a general decline in the quality of life, including permanent disability with high health care costs to patients, their families and the health care providers.

There have been a number of experimental approaches and targets evaluated to develop drugs for the protection of cells from degeneration. Glutamate, the main excitatory neurotransmitter in the central nervous system, is necessary for many normal neurological functions, including learning and memory. Overactivation of glutamate receptors, however, results in excitotoxic neuronal injury, has been implicated in the pathogenesis of neuronal loss in the central nervous system (CNS) following several acute insults, including hypoxia/ischemia. During brain ischemia caused by stroke or traumatic injury, excessive amounts of the excitatory amino acid glutamate are released from damaged or oxygen deprived neurons. This excess glutamate binds to the N-methyl-D-aspartate (NMDA) receptor which opens the ligand-gated ion channel thereby allowing calcium influx, producing a high level of intracellular calcium which activates biochemical cascades resulting in protein, DNA, and membrane degradation leading to cell death. This phenomenon, known as excitotoxicity, is also thought to be responsible for the neurological damage associated with other disorders ranging from hypoglycemia and cardiac arrest to epilepsy. In addition, there are preliminary reports indicating similar involvement in the chronic neurodegeneration of Huntington's, Parkinson's and Alzheimer's diseases. Accordingly, many pharmaceutical strategies have been assessed which aim to decrease levels of glutamate excess.

Oxidative stress, caused by reactive oxygen species, represents another injury mechanism implicated in many of the same acute and chronic diseases. Reactive oxygen species (e.g., superoxide radical) would cause oxidative damage to cellular components, such as peroxidation of cell membrane lipids, inactivation of transport proteins, and inhibition of energy production by mitochondria.

Glutamate excitotoxicity and oxidative stress, may be interlinked; reactive oxygen species formation may occur as a direct consequence of glutamate receptor overstimulation and thus mediate a component of glutamate. Excitotoxicity, in turn, can be reduced by free radical scavengers, including C, Zn-superoxide dismutase, the 21-aminosteroid "lazaroids", the vitamin E analog, trolox, spin-trapping agents such as phenylbutyl-N-nitrone, and the ubiquinone analog, idebenone which reduce the amount of reactive oxygen species.

Mooradian has reported that certain estrogens have significant anti-oxidant properties in *in vitro* biochemical assays, but that this effect is not seen with all estrogens. (See, J. Steroid Biochem. Molec. Biol., 45 (1993) 509-511.) Because of the variation in anti-oxidant properties noted by Mooradian in his biochemical assays, he concluded steroid molecules must have certain anti-oxidant determinants which were as yet unknown. Similar observations concerning steroids with phenolic A rings were reported in PCT Patent Application No. WO 95/13076, wherein biochemical assays were used to show anti-oxidant activity. However, the assays used by Mooradian, as well as those used in WO 95/13076, were biochemical assays and, as such, did not directly examine the effects of these molecules on cells. In contrast, Simpkins et al. describe, in U.S. Patent No. 5,554,601 for example, cell-based assays to determine a method of conferring neuroprotection on a population of cells using estrogen compounds based on demonstrated cell protective effects. As a result, in recent years it has become recognized that estrogen, as well as other polycyclic phenols, may be used for this purpose. (See, e.g., U.S. Patent Nos. 5,972,923; 5,877,169; 5,859,001; 5,843,934; 5,824,672; and, 5,554,601.)

The mechanism by which estrogen compounds bring about a neuroprotective effect is still not fully understood. However, these compounds have been shown to have a number of different physiological and biochemical effects on neurons. For example, estrogen has been shown to stimulate the production of neurotrophic agents that in turn stimulate neuronal growth. Estrogen compounds have also been found to inhibit NMDA-induced cell death in primary neuronal cultures (see, e.g., Behl et al. Biochem. Biophys Res. Commun. (1995) 216:973; Goodman et al. J. Neurochem. (1996) 66:1836), and further to be capable of removing oxygen free radicals and inhibiting lipid peroxidation (see, e.g., Droescher et al. WO 95/13076). For example, Droeschler et al. describe cell free in vitro assay systems using lipid peroxidation as an endpoint in which several estrogens, as well as vitamin E, were shown to have activity. Estradiol has also been reported to reduce lipid peroxidation of membranes (see, e.g., Niki (1987) Chem. Phys. Lipids 44:227; Nakano et al. Biochem. Biophys. Res. Comm. (1987) 142:919; Hall et al. J. Cer. Blood Flow Metab. (1991)11:292). Other compounds, including certain 21-amino steroids and a glucocorticosteroid, have been found to act as anti-oxidants and have been examined for their use in spinal cord injury, as well as head trauma, ischemia and stroke. (See, e.g., Wilson et al. (1995) J. Trauma 39:473; Levitt et al. (1994) J. Cardiovasc. Pharmacol 23:136; Akhter et al. (1994) Stroke 25; 418).

While anti-oxidant behavior is believed to be an important property, a number of other factors are believed to be involved in achieving neuroprotection. As a result, it is to be noted that therapeutic agents selected on the basis of a single biochemical mechanism may have limited generalized utility in treating disease or trauma in patients. For example, in order to achieve an anti-oxidant effect *in vitro* using estrogen, Droescher et al. used very high doses of estrogens. Such doses, even if effective on neurons *in vivo,* would have limited utility in treating chronic neurological conditions because of associated problems of toxicity that result from the prolonged use of these high dosages.

In addition to the issues related to compound toxicity, consideration must also be given to the ability of a particular compound to reach the target site, which in some applications is controlled by the ability of the compound to cross the blood-brain barrier. The blood-brain barrier is a complex of morphological and enzymatic components that retards the passage of both large and small charged molecules, and thus limits the access of such molecules to cells of the brain. Furthermore, not only must the compound be capable of reaching the target site, but it must also do so in a state or configuration which enables it to carry-out its designated function.

In view of the foregoing, it can be seen that a need continues to exist for the identification of compounds which have demonstrated biological efficacy in protecting humans from the consequences of abnormal cell death in body tissue; compounds which are capable of crossing the blood-brain barrier and which are suitable for administration in dosages which are non-toxic. This identification requires continuing advances in the understanding of the structural requirements for compositions capable of inducing neuroprotection, which in turn provide the basis for designing novel drugs that have enhanced cytoprotective properties while at the same time have reduced adverse side effects.

Lunn et al "The Adamantyl Carbonium Ion as a Dehydrogenating Agent, its Reactions with Estrone" Tetrahedron, vol. 24, 1968 pages 6773 to 6776, describes the dehydrogenation of estrone with adamantly carbonium ion. With modified conditions both t-butyl and adamantly carbonium ion sources gave 2-substituted estrone derivatives. Miller et al "In Vitro Antioxidant Effects of Estrogens with a Hindered 3-OH Function on the Copper-induced Oxidation of Low Density Lipoprotein" Steriods, vol. 61, 1996 pages 305 to 308, discloses estrogens with some bulky alkyl substituents in both the 2- and 4-positions. These are evaluated for the ability to inhibit the *in vitro* oxidation of low density lipoprotein as determined by the thiobarbituric reactive substances method.

### SUMMARY OF THE INVENTION

The present invention relates, in one aspect, to a compound having cytoprotective activity for use in the treatment of a cytodegenerative disease, the compound having the formula: 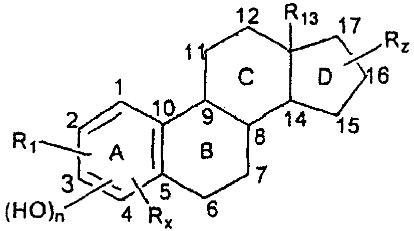 wherein: n is 1 or 2; R¹ is t-butyl; R^{x} is hydrogen or alkyl; R¹³ is hydrogen or alkyl; and, R^{z} is hydrogen, hydroxy, alkyl, or oxo,

The present invention relates in another aspect to a compound having the formula: 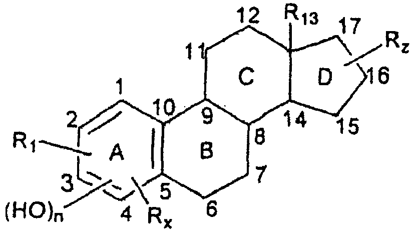 wherein: n is 1 or 2; R¹ is t-butyl; R^{x} is hydrogen or alkyl; R¹³ is hydrogen or alkyl; and, R^{z} is hydrogen, hydroxy, alkyl, or oxo, with the proviso that the compound does not having one of the following formulas:

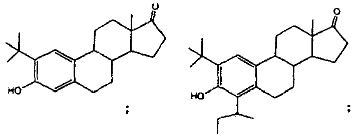

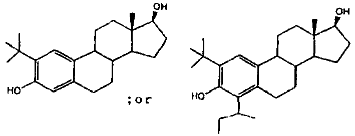

### BRIEF DESCRIPTION OF THE DRAWINGS

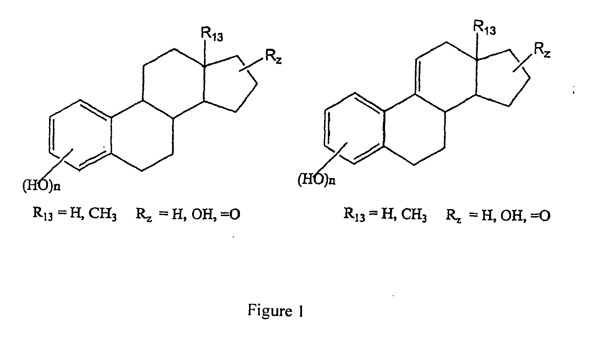

Figure 1 generally illustrates chemical structures of some preferred hydroxy-substituted aromatic compounds (e.g., phenols, catechols, etc., wherein n = 1 or 2), which can be modified with a tertiary-butyl substituent as described herein in accordance with the present invention and which may be used to confer cytoprotection to a population of cells upon the administration of an effective dose thereof.

### DETAILED DESCRIPTION OF THE PREFERRED EMBODIMENTS

It is now recognized that certain polycyclic phenolic compounds, in particular estrogen-based compounds, have cytoprotective, and in some cases neuroprotective, activity (see, e.g., U.S. Patent Nos. 5,972,923; 5,877,169; 5,859,001; 5,843,934; 5,824,672; 5,554,601; 6,197,833; and, 6,207,658). Without being held to a particular theory, it is generally believed that the activity associated with estrogen compounds is, at least in part, a result of the ability of estrogens, or more generally polycyclic phenolic compounds, because of their lipophilic nature, to become inserted into the cell membrane. Once in this position, the intact phenol group can donate a hydroxy hydrogen radical to prevent the cascade of membrane lipid peroxidation. Furthermore, it is generally believed that the significant potency of estrogens is because of their ability to donate a hydroxy hydrogen radical from several positions on the A ring (see, e.g., U.S. Patent No. 5,972,923), and because a relatively stable, oxidized estrogen is formed as a result of this hydrogen radical donation (due to the effects of resonance stability).

Surprisingly, it has now been discovered that these compounds, as well as dihydroxy (e.g., catechol), trihydroxy, etc. analogs thereof, may be modified by means of attaching a large or bulky hydrophobic substituent on the hydroxy-substituted ring, or alternatively at some other position proximate to the hydroxy group, yielding compounds which are also capable of conferring cytoprotection to a population of cells (e.g., neurons). In fact, Applicant's experimental data suggests the addition of such substituents, such as for example tertiary-butyl substituents, can act to enhance the cytoprotective activity of these compounds, relative to their respective non-substituted analogs.

### Modified Phenolic or Catecholic Compounds

As stated above, contrary to expectations, it has been discovered that previously reported phenolic compounds, as well as dihydroxy (e.g., catecholic) analogs thereof, can be modified by means of attaching a tertiary-butyl substituent, on or proximate to the hydroxy-bearing ring, in order to obtain a novel class of compounds having enhanced cytoprotective, and in some cases neuroprotective, activity. More specifically, it has been discovered that compounds having the formula (I):

X-R¹ (I)

wherein X generally represents the core or central structure, which in one exemplary embodiment is a phenol (such as those disclosed in, for example, U.S. Patent Nos. 5,972,923; 5,877,169; 5,859,001; 5,843,934; 5,824,672; 5,554,601; 6,197,833; and, 6,207,658; and in a second exemplary embodiment is a catechol (such as the dihydroxy analogs of those molecules disclosed in the aforementioned U.S. patents), to which the modifying hydrophobic substituent R¹ is attached, are suitable for use in treatments that protect a population of cells from cell death resulting from episodes of, for example, disease, trauma, isolation and removal of tissues or cells from the body, or exposure to toxins.

Referring now to Figure 1, examples of core structure, X, suitable for use in the present invention include compounds having a terminal hydroxy-bearing ring and at least three additional carbon rings (e.g., 3,17α-estradiol; 3,17β- estradiol; estratriene-3-ol; 2-hydroxy-17α-estradiol; 2-hydroxy-17β-estradiol; estrone, 2-hydroxy estrone; estriol; 2-hydroxy estriol; ethynyl estradiol; and, 2-hydroxy ethynyl estradiol).

It is to be noted in this regard that the above-referenced listing of compounds is not intended to be exhaustive. For example, the position of the hydroxy group or groups on the terminal ring is not, in all cases, narrowly critical; that is, in some cases, the hydroxy group may occupy essentially any available position (which, depending upon the particular structure of X, may be the 1, 2, 3, 4, etc. position on the terminal ring). Additionally, in some cases, it may be favorable for X to contain additional double bonds in conjugation with the hydroxy-aromatic ring (such as in the case of distilbesterol compounds), for example as in the case of polycyclic compounds having the general structure (IV): 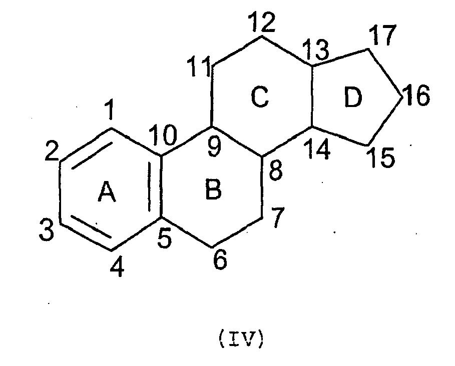 wherein a carbon-carbon double bond is present between C-6 and C-7, C-8 and C-9, C-9 and C-11, or one of the possible combinations thereof.

Without being held to any particular theory, it is generally believed this additional conjugation is favorable because it allows for the formation of a more stable, oxidized form of the compound; that is, it allows for additional delocalization of the phenoxy radical, which is believed to be formed as a result of the loss of a hydrogen radical to quench hydroperoxides (formed by the interaction of oxygen radical species with unsaturated fatty acids). Accordingly, X may be other than herein described without departing from the scope of the present invention.

In accordance with the present disclosure, it has been discovered that the core structure, X, may be modified by the addition of one or more large, hydrophobic substituents, R¹, in order to achieve enhanced cytoprotective activity, relative to the non-substituted analogs thereof (as further discussed and illustrated herein). More specifically, it has been found that the cytoprotective activity of, for example, the above-described phenolic compounds (such as those described by Simpkins et al.), as well as the dihydroxy-analogs thereof, can be enhanced by the attachment of such a substituent on the terminal, hydroxy-bearing ring.

Generally speaking, it has been found that enhanced cytoprotective activity is achieved when, in one embodiment, R¹ is a tertiary-butyl substituent.

Without being held to a particular theory, this enhanced activity is believed to be, at least in part, a result of the hydrophobic substituent R¹ taking up a position within the void region of the cell membrane or lipid bilayer, thus acting to "anchor" the compound and orient it such that the hydroxy-bearing aromatic ring (e.g., phenol or catechol) is positioned proximate double bonds in the fatty acids of the lipid chains. The enhancement in the effectiveness or activity of the present compounds is therefore believed to be a result of the fact that, because of their composition and structure, these compounds are naturally positioned within the environment to which they are delivered at a location which optimizes their effectiveness.

Furthermore, it is generally believed that orientation of the present compounds within the lipid bilayer or cell membrane may be further aided, in some cases, by the addition or attachment of a polar or hydrophilic group at or near the end of the compound which is substantially opposite the end to which the hydrophobic substituent R¹ is attached. Other factors which may also impact orientation of the compound include: (i) a substantially planar core structure X, and additionally the entire compound, (which is believed to enhance the performance of the present compounds); and/or (ii) the absence of a polar or hydrophilic substituent (R², R³, etc.) at a centrally located position on the compound (which is believed to detrimentally impact performance). For example, in the case of estrogen-like compounds, Applicant's experience to-date suggests changing the stereochemistry on the B or C ring by, for example, opening the B ring decreases activity, and the presence of a polar group on the B or C ring reduces activity as well.

Accordingly, essentially any large, hydrophobic substituent which achieves the desired result (i.e., which acts to "anchor" the compound and orient it at an optimal position within the lipid bilayer or cell membrane) may be employed in accordance with the present invention. More specifically, it is generally believed that essentially any substituent may be employed provided it is: (i) sufficiently hydrophobic in nature, such that it will be "dawn" or "pulled" into the hydrophobic portion of the bilayer or membrane; (ii) sufficiently large, such that once in the hydrophobic region it disturbs fatty acids in the membrane, thus disrupting membrane integrity to a degree which results in the substituent being forced into the membrane void; and, (iii) sufficiently long, either by itself or by the use of a linker, such that the hydroxy-substituted ring is positioned proximate the fatty acid double bonds.

With respect to the position of the hydroxy-substituted ring, it is to be noted that a typical membrane phospholipid has a length ranging from about 20 to about 30Å (angstroms),

as measured from about the end of the polar head group to about the end of the C-16 alkyl chain (by means of molecular modeling programs standard in the art). Accordingly, it is to be noted that in such cases essentially any combination of (i) a terminal hydroxy-bearing aromatic ring structure (e.g., phenol or catechol), and (ii) a hydrophobic substituent on or proximate to the hydroxy group on the aromatic ring structure, may be employed in the present invention, provided the distance between about the end of the hydrophobic "anchoring" substituent R¹ and about the opposite end of the hydroxy-bearing ring ranges from about 10 to less than about 30Å (i.e., about 15Å, 20Å, 25Å).

It is to be further noted that, as previously mentioned, while the "anchoring" substituent is typically attached directly to hydroxy-bearing aromatic ring, it may also be attached proximate this hydroxy group or groups; that is, the point of attachment of this substituent is not narrowly critical in all applications, provided that in such applications the point of attachment of the substituent is sufficient to orient the compound in such a way that the desired enhancement in activity is achieved. Accordingly, the substituent R¹ may be 1, 2, 3, 4 or more carbons from the hydroxy-bearing carbon, in some cases. Typically, however, the substituent R¹ will be adjacent or alpha to the hydroxy-bearing carbon. For example, in certain preferred embodiments, the hydroxy group is at a 2 or 3 position, which means R¹ is preferably in a 2, 3 or 4 position.

It is to be still further noted that, as illustrated below, the anchoring substituent R¹ may be directly attached to the core molecule or it may alternatively be attached via a linker ("L"), provide the linker is of a length sufficient to generally position the hydroxy-bearing aromatic ring structure as described above.

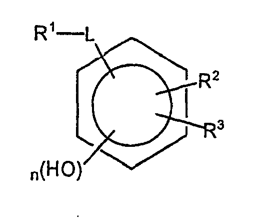

Typically, a hydrocarbylene linker (e.g., alkyl, akenyl, alkynyl) will be employed having a length ranging from about 1 to about 6 carbons in the main chain (e.g., methylene, ethylene, ethenylene, propylene, propenylene, etc.), with 1, 2 or 3 carbons in the main chain being preferred in some instances. Alternatively, however, in some embodiments, a hetero-substituted hydrocarbylene linker (such as an ether or thioether) may be employed.

### Additional and/or Alternative Substitution

In addition to the presence of one or more hydrophobic substituents, R¹, as described herein, it is to be noted that one or more other substituents (e.g., R², R³, R^{x}, etc.), which may be the same or different, may be attached to the hydroxy-bearing aromatic ring, or alternatively to some other segment or portion of the core structure X, provided the hydroxy-bearing ring remains in a substantially terminal position in the overall compound (i.e., X-R¹) structure; that is, it is to be noted that the present compound may optionally contain one or more additional substituents, which may be the same or different, at various available positions on the core structure, X, as illustrated below wherein X is a polycyclic structure (e.g., an estrogen derivative) having the formula (V):

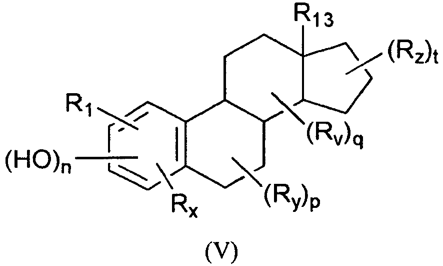

These other substituents (e.g., R^{y}, R^{v}, R^{z}) may, in some embodiments, be independently selected from the group consisting of hydrogen, substituted or unsubstituted hydrocarbyl (e.g., methyl, ethyl, propyl, propenyl, butyl, etc.), halogens (e.g., fluoro, bromo, chloro), amides, sulfates, and nitrates (wherein p, q and t generally represent the number of such substituents present on, in this case, the B, C and D rings respectively, and which typically range from 0 to 2 for the B and C rings, and 0 to 3 for the D ring).

Furthermore, as previously noted, the substituents (e.g., R² and R³) may, in combination with the carbon atoms to which they are bound, form a second, fused ring (e.g., a 5, 6, 7, etc. membered hydrocarbyl or heterohydrocarbyl) structure with the terminal, hydroxy-bearing ring. This second ring may, in turn, be further substituted in the same way as described herein with reference to the terminal hydroxy-bearing ring; that is, the second ring may have one or more substituents (e.g., R⁴, R⁵, etc.) independently selected from the group provided for R² and R³, which means X may in some embodiments comprise a third, forth, fifth, etc. ring structure (as shown, for example, in Figure 1, and as further described herein).

In some preferred embodiments, substituents R², R³, R⁴, R⁵, etc., (as well as R^{y}, R^{v}, R^{z}) may be, for example:
(a) Alkyl, alkenyl, alkynyl, containing up to about six carbon atoms in the main chain or ring structure (e.g., methyl, ethyl, propyl, butyl, pentyl, hexyl, dimethyl, isobutyl, isopentyl, tert-butyl, sec-butyl, methylpentyl, neopentyl, isohexyl, ethenyl, propenyl, butenyl, pentenyl, hexenyl, hexadienyl, 1,3-hexadiene-5-ynyl, isopropenyl, ethynyl, ethylidenyl, vinylidenyl, isopropylidenyl); sulfate; mercapto; methylthio; ethylthio; propylthio; methylsulfinyl; methylsulfonyl; thiohexanyl; thiopentyl; thiocyanato; sulfoethylamido; thionitrosyl; thiophosphoryl; p-toluenesulfonyl; amino; imino; cyano; carbamoyl; acetamido; hydroxyamino; nitroso; nitro; cyanato; selecyanato; arccosine; pyridinium; hydrazide; semicarbazone; carboxymethylamide; oxime; hydrazone; sulfurtrimethylammonium; semicarbazone; O-carboxymethyloxime; aldehyde hemiacetate; methylether; ethylether; propylether; butylether; benzylether; methylcarbonate; carboxylate; acetate; chloroacetate; trimethylacetate; cyclopentylpropionate; propionate; phenylpropionate; carboxylic acid methylether; formate; benzoate; butyrate; caprylate; cinnamate; decylate; heptylate; enanthate; glucosiduronate; succinate; hemisuccinate; palmitate; nonanoate; stearate; tosylate; valerate; valproate; decanoate; hexahydrobenzoate; laurate; myristate; phthalate; hydroxy; ethyleneketal; diethyleneketal; chloroformate; formyl; dichloroacetate; keto; difluoroacetate; ethoxycarbonyl; trichloroformate; hydroxymethylene; epoxy; peroxy; dimethyl ketal; acetonide; cyclohexyl; benzyl; phenyl; diphenyl; benzylidene; and, cyclopropyl. The substituent(s) may in some embodiments be attached to any of the constituent rings of X (i.e., the hydroxy-substituted ring or another ring bound or fused thereto) to form, for example, a pyridine, pyrazine, pyrimidine, or v-triazine. The substituent(s) may also include, for example, any of the six member or five member rings in section (b), below.
(b) A cyclic or heterocyclic carbon ring, which may be an aromatic or non-aromatic ring and which may be bound (directly or via a linker) or bused with the hydroxy-substituted substituted ring. This cyclic or heterocyclic ring may optionally be substituted with any substituent described in (a) above. This additional ring structure, alone or in combination with the hydroxy-substituted A-ring, may be selected from, for example, one or more of the following structures: phenanthrene; naphthalene; napthols; diphenyl; benzene; cyclohexane; 1,2-pyran; 1,4-pyran; 1,2-pyrone; 1,4-pyrone; 1,2-dioxin; 1,3-dioxin (dihydro form); pyridine; pyridazine; pyrimidine; pyrazine; piperazine; s-triazine; as-triazine; v-triazine; 1,2,4-oxazine; 1,3,2-oxazine; 1,3,6-oxazine (pentoxazole); 1,2,6-oxazine; 1,4-oxazine; o-isoxazine; p-isoxazine; 1,2,5-oxathiazine; 1,2,6-oxathiazine; 1,4,2-oxadiazine; 1,3,5,2-oxadiazine; and morpholine (tetrahydro-p-isoxazine). Additionally, any of the above carbon ring structures may be linked directly or via a linkage group to a heterocyclic aromatic or nonaromatic carbon ring, such as: furan; thiophene (thiofuran); pyrrole (azole); isopyrrole (isoazole); 3-isopyrrole (isoazole); pyrazole (1,2-daizole); 2-isoimidazole (1,3-isodiazole); 1,2,3-triazole; 1,2,4-triazole; 1,2-diothiole; 1,2,3-oxathiole, isoxazole (furo(a)monozole); oxazole (furo(b)monazole); thiazole; isothiazole; 1,2,3-oxadiazole; 1,2,4-oxadiazole; 1,2,5-oxadiazole; 1,3,5-oxadiazole; 1,2,3,4-oxatriazole; 1,2,3,5-oxatriazole; 1,2,3-dioxazole; 1,2,4-dioxazole; 1,3,2-dioxazole; 1,3,4-dioxazole; 1,2,5-oxathiazole; 1,3-oxathiole; and, cyclopentane. These compounds in turn may have associated substituent groups selected from section (a) or section (b) that are substituted on the ring at any of the available sites.

The core hydroxy-substituted structure, X, of the present invention may be a cyclopentanophen(a)anthrene ring compound, for example selected from the group consisting of the hydroxy-substituted analogs of: 1,3,5(10),6,8-estrapentaene; 1,3,5(10),6,8,11-estrahexaene; 1,3,5(10),6,8,15-estrahexaene; 1,3,5(10),6-estratetraene; 1,3,5(10),7-estratetraene; 1,3,5(10),8-estratetraene; 1,3,5(10),16-estratetraene; 1,3,5(10),15-estratetraene; 1,3,5(10)-estratriene; and 1,3,5(10),9(11)-estratetraene.

The present disclosure is further directed to any compound as described herein, as well as to the administration thereof to treat a cytodegenerative disease, including precursors or derivatives selected from raloxifen, tamoxifen, androgenic compounds, as well as their salts, where an intact hydroxy-bearing aromatic ring is present, with a hydroxy group present on carbons 1, 2, 3 and 4 of the terminal phenol ring.

These compounds may be in the form of a prodrug, which may be metabolized to form an active polycyclic phenolic, or catecholic, compound having cytoprotective, and in some cases neuroprotective, activity.

With respect to additional substituents (e.g., R², R³, R⁴, R⁵, R^{x}, R^{y}, R^{v}, R^{z}, etc.), it is to be noted that when such additional substituents are present on the terminal, hydroxy-substituted aromatic ring, or proximate thereto, in some embodiments it is preferred that these substituents be small (e.g., methyl, ethyl, propyl, butyl), relative to the size of the R¹ substituent of the present invention. Stated another way, it is preferred that in some embodiments of the present disclosure only one large, hydrophobic group be attached to the hydroxy-bearing aromatic ring, or proximate thereto.

Additionally, it is to be noted that when a smaller substituent is present, it is preferred that this substituent likewise be hydrophobic in nature. Again, without being held to a particular theory, it is generally believed that the presence of a hydrophilic group may interfere with the position and/or orientation of the overall compound within the cell membrane or lipid bilayer.

Finally, it is to be noted that substituent R¹ may itself be substituted. For example, when R¹ is tertiary butyl, a carbon atom thereof, may optionally be substituted to increase the hydrophobicity (such as by attaching a halogen).

### Additional Preferred Embodiments

### 1. Modification of the Hydroxy-substituted Aromatic Ring Structure

As previously noted, the core or terminal hydroxy-substituted aromatic ring structure may optionally be modified with one or more substituents in addition to the R¹ substituent. In one preferred embodiment, the hydroxy-substituted aromatic ring is additionally modified with a substituted or unsubstituted hydrocarbyl (e.g., methyl, ethyl, propyl, butyl, etc.) substituent. More preferably, when R¹ is a tertiary-butyl substituent, the hydroxy-substituted aromatic ring is additionally modified with an alkyl substituent (such as methyl, ethyl, propyl, methylpropyl, etc). Even more preferably, these substituents are alpha or adjacent to the hydroxy group on the aromatic ring; that is, preferably the hydroxy group is between the two substituents, the two substituents occupying carbon atoms which are directly bound to the carbon atom occupied by the hydroxy group.

In a preferred embodiment, the hydroxy-substituted ring may be modified with two substituents, such as t-butyl/methyl or ethyl.

### 2. Polycyclic, Hydroxy-substituted Aromatic Ring Structure

As previously noted, the core hydroxy-substituted aromatic ring structure may be part of a larger, polycyclic core structure (e.g., bicyclic, tricyclic, tetracyclic, etc.). In one preferred embodiment, the hydroxy-substituted core structure has the formula (VI): 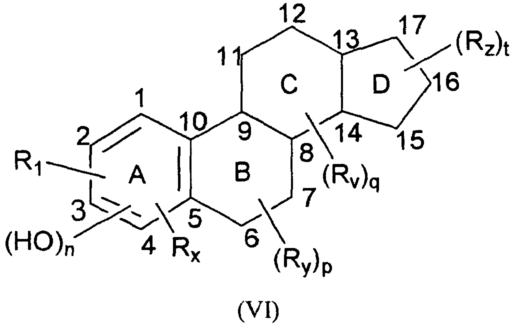 wherein n is typically 1 or 2, R¹ is a t-butyl substituent, the hydroxy-substituted ring being the terminal or A-ring of the structure, and R^{x}, R^{y}, R^{v} and R^{z} are as defined herein. More preferably, however, the compound of the present invention has the formula (VII): 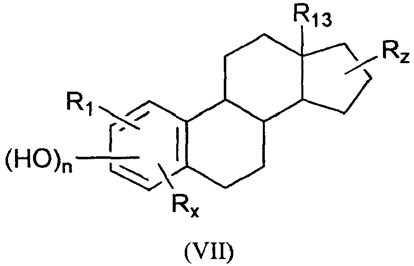 wherein: n is as defined above, R¹ is a t-butyl substituent; R^{x} is selected from the group consisting of hydrogen and alkyl; R¹³ is hydrogen or alkyl; and, R^{z} is one or more substituents selected from hydrogen, hydroxy, alkyl, or oxo (R^{y}, R^{v} being hydrogen). Some of the preferred combinations of such substituents include:

| **R¹** | **R^{x}** | **R^{z}** |
|---|---|---|
| t-butyl | hydrogen | hydroxyl |
| t-butyl | hydrogen | oxo |
| t-butyl | methyl | oxo |

wherein for example R¹, R^{x} and R^{z} occupy the C-2, C-4 and C-17 positions on the ring, respectively (R^{z} being in the alpha or beta position when is it hydroxy, for example).

In this regard it is to be noted that the present disclosure encompasses a number of compounds having one or more chiral centers therein. Generally speaking, therefore, it is to be understood that the configuration at one or more of these chiral centers may change without departing from the scope of the intended disclosure; that is, it is to be understood that the present disclosure extend to compounds specifically or generally described herein, as well as all related diastereomers and enantiomers (e.g., (i) the naturally-occurring estrogen configuration wherein, when present, substituents at the C-8, C-9, C-13 and C-14 position are beta, alpha, beta and alpha, respectively, or (ii) the non-naturally-occurring estrogen configuration wherein, when present, these substituents have the alpha, beta, alpha, beta configuration).

### Administration/Application

Generally speaking, the process of the present disclosure involves the treatment of a population of cells in a subject (e.g., animal or human), in order to confer cytoprotection to that population, by the administration of an effective dose of the above-described compound. Experience to-date suggests such protection can be achieved at low plasma concentrations, concentrations which can be significantly lower than those needed for the non-substituted (i.e., non-R¹ substituted) analogs of the present compounds. More specifically, a cytoprotective or even a neuroprotective effect can be achieved, in some cases, at plasma concentrations of less than about 10 µM, 1 µM, 500 nM, 100 nM, 10 nM, or even 1 nM (i.e., from about 0.1 nM to about 1 nM).

Administration of any of the compounds of the disclosure may be achieved by means standard in the art, and may include the use of a single compound or a mixture of cytoprotective compounds, their enantiomers or diastereomers, or pharmaceutically acceptable salts thereof. The recommended route of administration of the compounds of the present disclosure includes oral, intramuscular, transdermal, buccal, nasal, intravenous and subcutaneous. Methods of administering the compounds of the invention may be by dose or by controlled release vehicles.

Additionally, it is to be noted that, similar to the approach described by Simpkins et al. in U.S. Patent No. 5,972,923, a pharmaceutical preparation may also include, in addition to one or more compounds of the present disclosure, an additional antioxidant. As noted by Simpkins et al., in reference to compounds similar to those of the present invention, synergistic effects may be achieved in certain circumstances when such a combination is employed. For example, Simpkins et al. reports that estratrienes exhibit approximately a 1000-5000 fold enhancement in their cytoprotective effect when administered with the antioxidant, glutathione.

The present compounds are suitable, for example, in treating subjects suffering from trauma, chronic degenerative diseases or acute disease such as induced by an ischemic attack. Specific examples include Alzheimer's disease, Parkinson's disease, stroke, ischemia, heart attack or angioplasty, or brain or spinal cord trauma, hypoglycemia, anoxia, burns or surgeries that result in the loss of nutrient flow to the tissues. Other diseases that may be treatable with compounds of the current invention include: heart disease, including myocardial infarction, ophthalmologic diseases including macular degeneration, lens or retinal degeneration, formation of cataracts and glaucoma, alcoholism, alcohol withdrawal, drug-induced seizures vascular occlusion, epilepsy, cerebral vascular hemorrhage, hemorrhage; environmental excitotoxins, dementias of all type, drug-induced brain damage and other systemic or acute degenerative diseases characterized by necrotic or apoptotic cell death. To-date, there are no known cures and few therapies that slow the progression of these diseases. However, the present invention provides compounds which can be used as therapeutics or as prophylactics to treat, prevent or delay the onset of symptoms.

Certain embodiments of the present disclosure may further be applied to the procedure of tissue transplantation, prior, during or after removal or reperfusion of cells, tissues or organs or during storage of the cells, tissues or organs and is applicable to any of the cells in the body.

### Preparation

Generally speaking, the compounds of the present disclosure may be prepared by means standard in the art. Specific details for the preparation of certain preferred compounds are provided herein in the Examples, below.

### Activity

The activity of the compounds of the present invention may be determined by means standard in the art (see, e.g., U.S. Patent Nos. 5,972,923; 5,877,169; 5,859,001; 5,843,934; 5,824,672; and, 5,554,601). Alternative methods for determining activity are described in detail herein in the Examples, below.

### Definitions

As used herein, the following phrases or terms shall have the noted meanings; however, it is to be understood that these definitions are intended to supplement and illustrate, not preclude or replace, the definitions known to those of skill in the art.

"Hydroxy-substituted aromatic" or "hydyroxy-bearing aromatic" structure or ring, as well as variations thereof, refers to a terminal ring of a compound of the present disclosure which is both aromatic and substituted with one or more hydroxy groups. It is therefore to be understood that such phrases are intended to refer to compounds wherein the entire structure is aromatic (e.g., naphthalene, anthracene, and phenanthracene), as well as to compounds wherein only the terminal ring is aromatic (e.g., indan and 1,2,3,4-tetrahydronaphthlene).

"Cytoprotection" refers to the protection of cells against cell death or cell damage that would otherwise occur in the absence of a protective agent, where the cell death or cell damage might be caused by any mechanical damage, nutritional deprivation (including oxygen deprivation), trauma, disease processes, damage due to exposure to chemicals or temperature extremes, aging or other causes.

"Neuroprotection" is one form of cytoprotection and refers to the inhibition of the progressive deterioration of neurons that lead to cell death.

"Enhanced" cytoprotective or neuroprotective activity refers to the increase in activity of the compounds of the present invention (i.e., compounds having a large, hydrophobic substituent, R¹, attached), as compared to the non-substituted (i.e., non-R¹ substituted) analogs thereof.

"Non-fused, polycyclic" refers to polycyclic systems other than fused systems, and is intended to encompass bridged and spiro systems, as well as ring assemblies. (See, *e.g.*, Naming and Indexing of Chemical Substances for Chemical Abstracts, a reprint of Appendix IV from the Chemical Abstracts 1997 Index Guide, ¶¶147-155, pp. 260I-266I. )

"Fused systems" are polycyclic structures containing at least two rings of five or more members having (i) only "ortho" fusion, wherein adjoining rings have only two atoms in common and thus have n common faces and 2n common atoms, such as naphthalene, or (ii) "ortho" and "peri" fusion, wherein a ring has two, and only two, atoms in common with each of two or more rings, the total system containing n common faces and *fewer than 2n* common atoms, such as pyrene. (See, *e.g., Id.*)

"Bridged systems" are monocyclic or fused systems with valence bonds, atoms or chains connecting different parts of the structure.

"Spiro systems" have pairs of rings (or ring systems) with only one common atom. (See, *e.g., Id.*)

"Ring assemblies" have pairs of rings (or ring systems) connected by single bonds. (See, *e.g., Id.*)

An "estrogen compound" refers to any of the structures described in the 11th Edition of "Steroids" from Steraloids Inc., Wilton N.H. Included in this definition are isomers and enantiomers, including non-steroidal estrogens formed by modification or substitution of the compounds in the Steraloid reference. Other estrogen compounds included in this definition are estrogen derivatives, estrogen metabolites and estrogen precursors, as well as those molecules capable of binding cell-associated estrogen receptors as well as other molecules where the result of binding specifically triggers a characterized estrogen effect. Also included are mixtures of more than one estrogen, where examples of such mixtures are provided in, for example, U.S. Patent No. 5,972,923. Examples of α-estrogen structures having utility either alone or in combination with other agents are provided in, for example, U.S. Patent No. 5,972,923 as well.

A "non-estrogen compound" refers to a compound other than an estrogen compound as defined above.

The terms "17-E2," "β-estradiol," "17β-stradiol," "β-17-E2" "17β-E2," "E2," "17βE2," and "βE2," are intended to be synonymous. Similarly, the terms "α17-E2," "α-17-E2," "α-estradiol," "17α-estradiol," "17αE2," and "αE2," as defined here and in the claims, are intended to be synonymous and correspond to the α-isomer of 17 β-estradiol.

"E-3-ol" refers to estra-1,3,5(10)-trien-3-ol.

The terms "polycyclic phenolic compound," "polycyclic compounds" or "polycyclic phenols" as used herein are generally synonymous and are defined, for example, in U.S. Patent No. 5,859,001; the terms generally include any compound having a phenolic A ring and may contain 2, 3, 4 or even more additional ring structures exemplified by the compounds described herein.

A "steroid" refers to a compound having numbered carbon atoms arranged in a 4-ring structure (see, e.g., J. American Chemical Society, 82:5525-5581 (1960); and, Pure and Applied Chemistry, 31:285-322 (1972)).

A "cytodegenerative" disorder or disease refers to a disorder or disease related to cell death or cell damage, which might be caused by any mechanical damage, nutritional deprivation (including oxygen deprivation), trauma, disease processes, damage due to exposure to chemicals or temperature extremes, aging or other causes.

A "neurodegenerative disorder" or "neurodegenerative disease" refers to a disorder or disease in which progressive loss of neurons occurs either in the peripheral nervous system or in the central nervous system. Examples of neurodegenerative disorders include: chronic neurodegenerative diseases, such as Alzheimer's disease, Parkinson's disease, Huntington's chorea, diabetic peripheral neuropathy, multiple sclerosis, amyotrophic lateral sclerosis; aging; and acute neurodegenerative disorders including: stroke, traumatic brain injury, schizophrenia, peripheral nerve damage, hypoglycemia, spinal cord injury, epilepsy, and anoxia and hypoxia.

"Linker" embraces a saturated or partially unsaturated moiety, typically a hydrocarbylene (e.g., alkylene, akenylene, akynylene), or alternatively a hetero-substituted hydrocarbylene (e.g., wherein a carbon in the main chain has been substituted by a heteroatom, such as oxygen or sulfur), interposed between the core ring structure X and the modifying hydrophobic substituent, R¹, or alternatively between the core ring structure X and another substituent (e.g., R², R³, etc.).

"Hydrocarbyl" embrace moieties consisting exclusively of the elements carbon and hydrogen, in a straight or branched chain, or alternatively a cyclic structure, which may optionally be substituted with other hydrocarbon, halo (e.g., chlorine, fluorine, bromine) or hetero (e.g., oxygen, sulfur) substituents. These moieties include alkyl, alkenyl, alkynyl and aryl moieties, as well as alkyl, alkenyl, alkynyl and aryl moieties substituted with other aliphatic or cyclic hydrocarbon groups such as, for example, alkaryl, alkenaryl and alkynaryl.

The alkyl groups described herein are, in some embodiments, preferably lower alkyl containing from about 1 to about 6 carbon atoms in the principal chain. They may be straight or branched chains and include methyl, ethyl, propyl, isopropyl, butyl, isobutyl, tertiary butyl, pentyl, hexyl and the like.

The alkenyl groups described herein are, in some embodiments, preferably lower alkenyl containing from about 2 to about 6 carbon atoms in the principal chain. They may be straight or branched chains and include ethenyl, propenyl, isopropenyl, butenyl, isobutenyl, pentenyl, hexenyl, and the like.

The alkynyl groups described herein are, in some embodiments, preferably lower alkynyl containing from about 2 to about 6 carbon atoms in the principal chain. They may be straight or branched chain and include ethynyl, propynyl, butynyl, isobutynyl, pentynyl, hexynyl, and the like. They may be substituted with aliphatic or cyclic hydrocarbon moieties or hetero-substituted with the various substituents defined herein.

The term "cycloalkyl" is used herein to refer to a saturated cyclic non-aromatic hydrocarbon moiety having a single ring or multiple condensed rings. Exemplary cycloalkyl moieties include, for example, cyclopentyl, cyclohexyl, cyclooctanyl, etc.

The term "cycloalkenyl" is used herein to refer to a partially unsaturated (i.e., having at least one carbon-carbon double bond), cyclic non-aromatic hydrocarbon moiety having a single ring or multiple condensed rings. Exemplary cycloalkenyl moieties include, for example, cyclopentenyl, cyclohexenyl, cyclooctenyl, etc.

"Substituted cycloalkyl" and "substituted cycloalkenyl" refer to cycloalkyl and cycloalkenyl moieties, respectively, as just described wherein one or more hydrogen atoms to any carbon of these moieties is replaced by another group such as a halogen, alkyl, alkenyl, alkynyl, substituted alkyl, substituted alkenyl, substituted alkynyl, aryl, substituted aryl, cycloalkyl, cycloalkenyl, substituted cycloalkyl, substituted cycloalkenyl, heterocyclo, substituted heterocyclo, heteroaryl, substituted heteroaryl, alkoxy, aryloxy, boryl, phosphino, amino, silyl, thio, seleno and combinations thereof.

"Hydrophobic," as used in the context of the substituent attached to the hydroxy-substituted aromatic ring structure (i.e., R1), generally refers to this substituent's affinity for nonaqueous environments, and more specifically to its affinity for the hydrophobic region of a lipid bilayer upon introduction thereto.

"Terminal," as used in the context of the hydroxy-substituted aromatic ring structure, generally refers to the position of the ring relative to the rest of the molecule, the ring being located at or proximate one end of the molecule, such as in the case of the tetracyclic estrogen compounds (the hydroxy-substituted aromatic ring being the A ring of the compound).

The following Examples set forth one approach for preparing and testing compounds in accordance with the present invention. These Examples are intended to be illustrative of compounds preferred for certain embodiments only, as well as their respective activity in the protection of neuron. Generally speaking, however, it is understood that in many cases drugs which protect neurons are also active in protecting non-neuronal cells. Accordingly, in advancing the understanding of the structural requirements for compositions capable of inducing neuroprotection, these results in turn provide the basis for designing novel drugs that have enhanced cytoprotective properties, as well.

### EXAMPLE 1:

### Preparation of 2-(1,1-Dimethylethyl)-3-hydroxy-4-methylestra-1,3,5(10)-trien-17-one

To a suspension of the known 3-hydroxy-4-methylestra-1,3,5(10)-trien-17-one (ref. Kaneko et al., 1964; Chemical Abstracts Registry Number [68969-90-4], 30 mg, 0.11mmol) in 1 mL of anhydrous pentane and 0.5 mL of t-butanol, was added 0.03 mL of boron trifluoride diethyl etherate while cooling with an ice bath and stirring. After 20 min., the reaction was stirred at room temperature for 2.5 h. Ice was added and the solid that formed was filtered, washed with water and dried overnight in a vacuum desiccator. The crude product was purified by chromatography (silica gel eluted with 10% ethyl acetate in hexanes) and then recrystallized from methylene chloride-hexanes to give 20 mg of pure product (56% yield): m.p.190-192°C; ¹H NMR(CDCl₃) δ0.90 (s, 3H, CH₃), 1.42 (s, 9H, C(CH₃)₃), 2.13 (s, 3H, CH₃), 2.79-2.81 (m, 2H, CH₂), 7.16 (s, 1H, Ar-H); ¹³C NMR (CDCl₃) δ 11.06, 13.70, 21.44, 26.16, 26.60, 26.68, 27.54, 29.82 (3 x C), 31.55, 34.43, 35.84, 37.63, 44.52, 47.89, 50.34, 121.42, 131.28, 133.00, 133.55, 150.56, 221.45. (Anal. calc'd. for C₂₃H₃₂O₂: C, 81.13; H, 81.09. Found: C, 81.09; H, 9.59.)

### Example 2:

### Preparation of Ent-(17β)-2-(1,1-dimethylethyl)estra-1,3,5(10)-triene-3,17-diol

A suspension of the known ent-17 β -estradiol (ref. Green et al., 2001, Chemical Abstracts Registry Number [3736-22-9], 30 mg, 0.11 mmol) and 0.06 mL of 2-methyl-2-propanol (0.63 mmol) in 1 mL anhydrous pentane was stirred at room temperature 15 min. and then at 0 °C to -5oC for 20 min. To this suspension was added 0.07 mL boron trifluoride diethyl etherate over 1 min. and stirring was continued at 0 °C to

-5 °C for 20 min. The reaction was allowed to warm to room temperature and during this period (~15 min.) a yellow solid that stuck to the flask was formed. After stirring for 30 min. at room temperature, ice was added. The powder-like solid was filtered, washed with water and dried in a vacuum desiccator over night. The crude product was purified by chromatography (silica gel eluted with 18% ethyl acetate in hexanes) to get the pure product which was then crystallized from acetone-hexane. The pure product (20 mg, 55% yield) had: m.p.177-179 °C; [α](25, D) -91.33 (c=0.225, CHCl₃); ¹H NMR(CDCl₃) δ0.78 (s, 3H, CH₃), 1.40 (s, 9H, C(CH₃)₃), 2.74-2.75 (m, 2H, CH₂), 3.74 (t, J = 8.4 Hz, CHOH), 6.41 (s, 1H, Ar-H); 7.19 (s, 1H, Ar-H); ¹³C NMR(CDCl3) δ52.05, 135.33, 133.37, 131.81, 124.04, 116.55, 81.98, 60.44, 50.03, 44.23, 43.26, 38.98, 36.78, 34.47, 30.57, 29.74 (3 x C), 28.90, 27.22, 26.38, 23.12, 14.17, 11.07. (Anal. calc'd. for C₂₂H₃₂O₂: MS m/z 328(M⁺), 313.)

### EXAMPLE 3:

### Comparative Study of Compound Activity

### Method: HT-22 Cell Neuroprotection Assay

HT-22 cells (immortalized hippocampal neurons of murine origin) were maintained in DMEM media (Life Technologies, Inc., Gaitherburg, MD) supplemented with 10% charcoal-stripped FBS (HyClone Laboratories, Inc., Logan, UT) and 20 µg/mL gentamycin, according to standard culture conditions.

Cells were plated at a density of 5,000 cells/well in clear-bottomed Nunc 96-well plates (Fisher Scientific, Orlando, FL) and allowed to incubate overnight. Steroids dissolved in DMSO were added at concentrations ranging from 0.01-10 µM and were co-administered with glutamate (10 mM or 20 mM). DMSO was used at concentrations of 0.1 % vol/vol as a vehicle control and had no discernible effect on cell viability. After ~16 h of glutamate exposure, cells were rinsed with PBS, pH 7.4, and viability was assessed by the addition of 25 µM calcein AM (Molecular Probes, Inc., Eugene, OR) in PBS for 15 min at room temperature. Fluorescence was determined (excitation 485, emission 530) using a fluorescence FL600 microplate reader (Biotek, Winooski, VT). Cells that were lysed by addition of methanol were used for blank readings. All data were normalized to % cell death, as calculated by (control value - insult value)/control value x 100.

### Resul ts

Test results are presented in Tables 1 and 2, below.

**TABLE 1**

| | | | | | | |
|---|---|---|---|---|---|---|
| 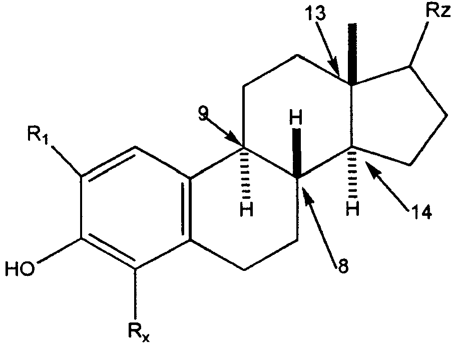 | | | | | | |

| | **Compound** | **R₁** | **Rₓ** | **R_{z}** | **Steroid concentration needed to protect 50% of neurons killed by 10 mM Glutamate ED₅₀ (µM)** | **Steroid concentration needed to protect 50% of neurons killed by 20 mM Glutamate D₅₀** (µM) |
|---|---|---|---|---|---|---|
| 3 | ZYC-15 | *t*-Butyl | H | β -OH | 0.045 | 0.14 |
| 7 | ZYC-14 | *t*-Butyl | H | =O | 0.25 | 0.30 |
| 9 | ZYC-25 | *t*-Butyl | Me | =O | 0.32 | 0.32 |
| 15 | 17- β Estradiol | H | H | β -OH | 2.21 | 3.01 |
| 16 | Estrone | H | H | =O | 3.03 | Not Done |
| 17 | 17 α -Estradiol | H | H | α -OH | 3.10 | 16.12 |

**TABLE 2**

| | | | | | | |
|---|---|---|---|---|---|---|
| 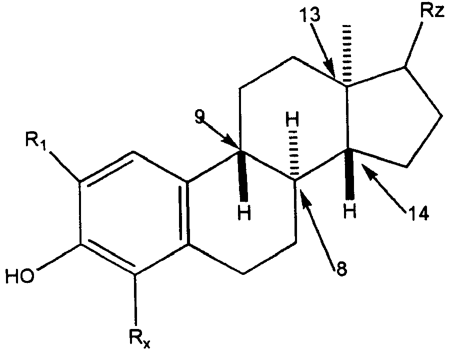 | | | | | | |

| | **Compound** | **R₁** | **Rₓ** | **R_{z}** | **Steroid concentration needed to protect 50% of neurons killed by 10 mM Glutamate ED₅₀ (µM)** | **Steroid concentration needed to protect 50% of neurons killed by 20 mM Glutamate ED₅₀ (µM)** |
|---|---|---|---|---|---|---|
| 2 | ZYC-34 | *t*-Butyl | H | α -OH | 0.24 | 0.26 |
| 3 | *Ent-*( 17 β)- H Estradiol | | H | α -OH | 1.07 | 1.27 |

In view of the above, it will be seen that the several objects of the invention are achieved. As various changes could be made in the above process and compound without departing from the scope of the invention which is defined by the claims appended hereto, it is intended that all matter contained in the above description be interpreted as illustrative and not in a limiting sense.

## Claims

1. A compound having cytoprotective activity for use in treatment of a cytodegenerative disease, the compound having the formula: 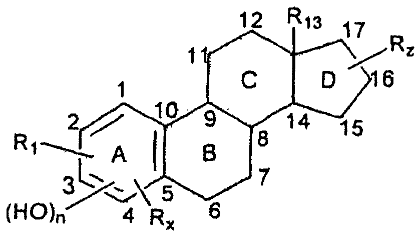 wherein: n is 1 or 2; R₁ is t-butyl; Rₓ is hydrogen or alkyl; R₁₃ is hydrogen or alkyl; and R_{z} is hydrogen, hydroxy, alkyl, or oxo.

2. The compound of Claim 1 having the formula: 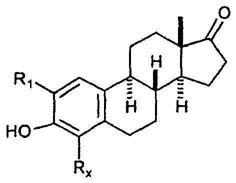 or 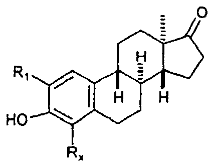 wherein R₁ and Rₓ are as defined in Claim 1.

3. The compound for use of Claim 1 having the formula: 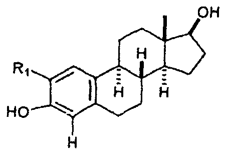 or 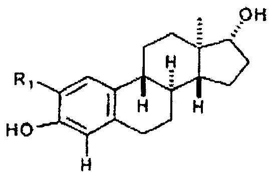 or 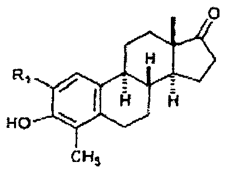 or

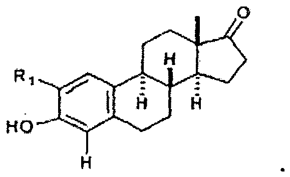

4. The compound for use of Claim 1 having the formula: 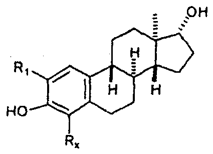 or the enantiomer thereof, wherein R₁ and Rₓ are as defined in Claim 1.

5. The compound for use of any one of Claims 1-4 wherein said treatment is of an animal having said disease.

6. The compound for use of any one of Claims 1-4 wherein said treatment is of a human having said disease.

7. The compound for use of any one of Claims 1-4 wherein said compound is administered to a population of cells to confer cytoprotection thereto.

8. The compound for use of Claim 7 wherein said cells are neurons.

9. The compound for use of Claim 7 wherein said cells are within ophthalmic tissue.

10. The Compound for use of any one of Claims 1-9 in combination with a pharmaceutically acceptable carrier, excipient or diluent.

11. The compound of any one of Claims 1-10 for use in the treatment of a cytodegenerative disease selected from Alzheimer's disease, Parkinson's disease, Huntington's disease, multiple sclerosis, amyotrophic lateral sclerosis, stroke, ischemia, ophthalmologic diseases, cerebral vascular hemorrhage, dementias, drug-induced brain damage and other systemic or acute diseases **characterized by** necrotic or apoptotic cell death.

12. The compound of Claim 11 for use in the treatment of ophthalmologic diseases selected from macular degeneration, lens or retinal degeneration and the formation of cataracts or glaucoma.

13. A compound having the formula: 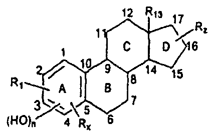 wherein: n is 1 or 2; R₁ is t-butyl; Rₓ is hydrogen or alkyl; R₁₃ is hydrogen or alkyl; and R_{z} is hydrogen, hydroxy, alkyl, or oxo, with the proviso that the compound does not have one of the following formulas:

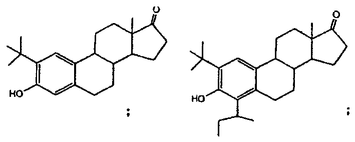

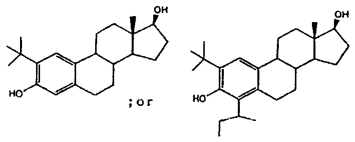

14. The compound of Claim 13 wherein said compound has the formula: 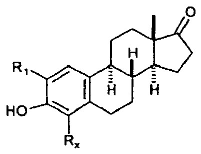 or 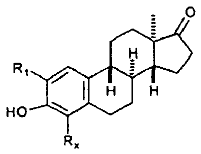 wherein R₁ is t-butyl, and Rx is methyl, ethyl, propyl, isopropyl, tertiary-butyl, pentyl or hexyl.

15. The compound of Claim 13 wherein the compound has the formula: 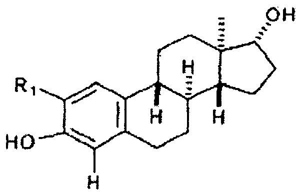 or

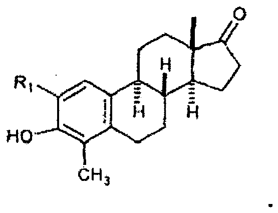

16. The compound of Claim 13 wherein the compound has the formula: 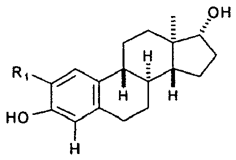 or the enantiomer thereof, wherein R₁ and Rₓ are as defined in Claim 13.

17. The compound of Claim 13 wherein Rₓ is alkyl, and selected from methyl, ethyl, propyl, isopropyl, methylpropyl, butyl, tertiary butyl, pentyl or hexyl.

18. The compound of Claim 13 wherein R₁₃ is alkyl, and selected from methyl, ethyl, propyl, isopropyl, methylpropyl, butyl, tertiary butyl, pentyl or hexyl.

19. The compound of Claim 13 wherein R_{z} is alkyl, and selected from methyl, ethyl, propyl, isopropyl, methylpropyl, butyl, tertiary butyl, pentyl or hexyl.

20. A compound of Claim 11 for use in the treatment of Parkinson's disease.

## Patentansprüche

1. Verbindung mit zellschützender Aktivität zur Verwendung in der Behandlung einer
zelldegenerativen Krankheit, wobei die Verbindung die Formel ; 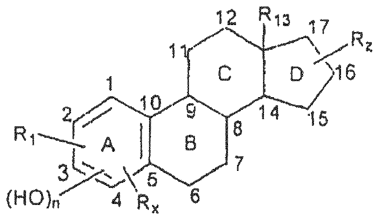 hat, wobei: n 1 oder 2 ist; R₁ t-Butyl ist; Rₓ Wasserstoff oder Alkyl ist; R₁₃ Wasserstoff oder Alkyl ist; und R_{z} Wasserstoff, Hydroxy, Alkyl oder Oxo ist.

2. Verbindung zur Verwendung nach Anspruch 1 mit der Formel: 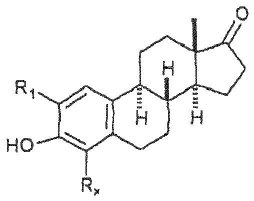 oder 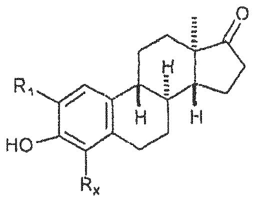 wobei R₁ und Rₓ wie in Anspruch 1 definiert sind.

3. Verbindung zur Verwendung nach Anspruch 1 mit der Formel: 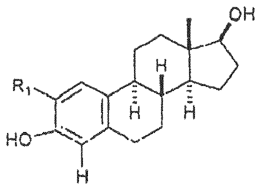 oder 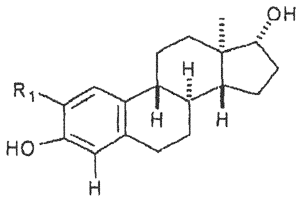 oder 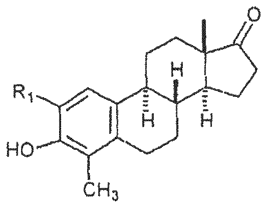 oder

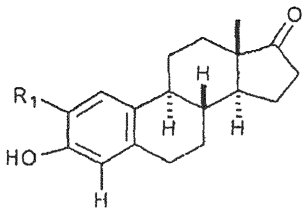

4. Verbindung zur Verwendung nach Anspruch 1 mit der Formel: 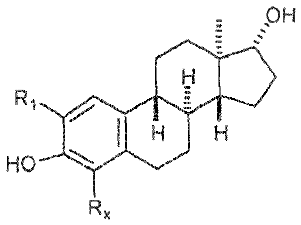 oder das Enantiomer davon, wobei R₁ und Rₓ wie in Anspruch 1 definiert sind.

5. Verbindung zur Verwendung nach einem der Ansprüche 1-4, wobei die Behandlung von einem Tier ist, das diese Krankheit hat.

6. Verbindung zur Verwendung nach einem der Ansprüche 1-4, wobei die Behandlung von einem Menschen ist, der diese Krankheit hat.

7. Verbindung zur Verwendung nach einem der Ansprüche 1-4, wobei die Verbindung einer Zellpopulation verabreicht wird, um ihr Zellschutz zu verleihen.

8. Verbindung zur Verwendung nach Anspruch 7, wobei die Zellen Nervenzellen sind.

9. Verbindung zur Verwendung nach Anspruch 7, wobei die Zellen in Augengewebe sind.

10. Verbindung zur Verwendung nach einem der Ansprüche 1-9 in Kombination mit einem pharmazeutisch annehmbaren Träger, Füllstoff oder Verdünnungsmittel.

11. Verbindung nach einem der Ansprüche 1 - 10 zur Verwendung in der Behandlung einer zelldegenerativen Krankheit ausgewählt aus Alzheimer-Krankheit, Parkinson-Krankheit, Huntington-Krankheit, Multipler Sklerose, amyotropher Lateralsklerose, Schlaganfall, Ischämie, Augenkrankheiten, Hirngefäßblutung, Demenzen, arzneimittelinduzierter Hirnschädigung und anderen systemischen oder akuten Krankheiten, die durch nekrotischen oder apoptotischen Zelltod gekennzeichnet sind.

12. Verbindung nach Anspruch 11 zur Verwendung in der Behandlung von Augenkrankheiten ausgewählt aus Makuladegeneration, Linsen- oder Netzhautdegeneration und der Bildung von Katarakten oder Glaukomen.

13. Verbindung mit der Formel: 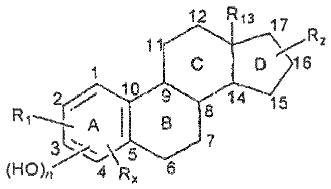 wobei: n 1 oder 2 ist; R₁ t-Butyl ist; Rₓ Wasserstoff oder Alkyl ist; R₁₃ Wasserstoff oder Alkyl ist; und R_{z} Wasserstoff, Hydroxy, Alkyl oder Oxo ist mit der Maßgabe, dass die Verbindung keine der folgenden Formeln hat:

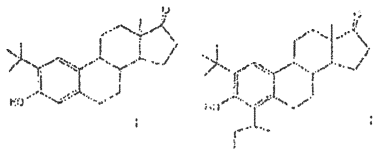

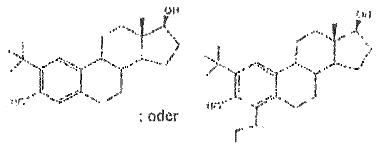

14. Verbindung nach Anspruch 13, wobei die Verbindung die Formel 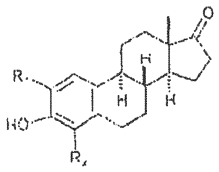 oder 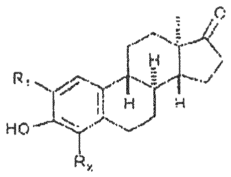 hat, wobei R₁ t-Butyl ist, und Rₓ ethyl, Ethyl, Propyl, Isopropyl, tertiäres Butyl, Pentyl oder Hexyl ist.

15. Verbindung nach Anspruch 13, wobei die Verbindung die Formel: 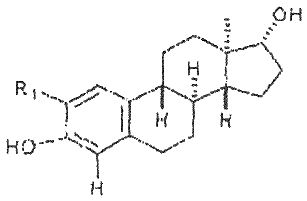 oder 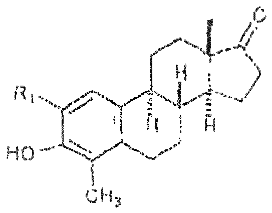 hat.

16. Verbindung nach Anspruch 13, wobei die Verbindung die Formel : 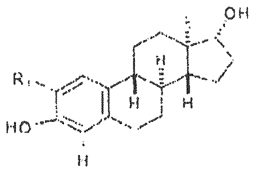 hat oder das Enantiomer davon, wobei R₁ und Rₓ wie in Anspruch 13 definiert sind.

17. Verbindung nach Anspruch 13, wobei Rₓ Alkyl ist und ausgewählt ist aus Methyl, Ethyl, Propyl, Isopropyl, Methylpropyl, Butyl, tertiärem Butyl, Pentyl oder Hexyl,

18. Verbindung nach Anspruch 13, wobei R₁₃ Alkyl ist und ausgewählt ist aus Methyl, Ethyl, Propyl, Isopropyl, Methylpropyl, Butyl, tertiärem Butyl, Pentyl oder Hexyl.

19. Verbindung nach Anspruch 13, wobei R_{z} Alkyl ist und ausgewählt ist aus Methyl, Ethyl, Propyl, Isopropyl, Methylpropyl, Butyl, tertiärem Butyl, Pentyl oder Hexyl,

20. Verbindung nach Anspruch 11 zur Verwendung in der Behandlung von Parkinson-Krankheit.

## Revendications

1. Composé ayant une activité cytoprotectrice, à utiliser dans le traitement d'une maladie cytodégénérative, le composé ayant la formule: 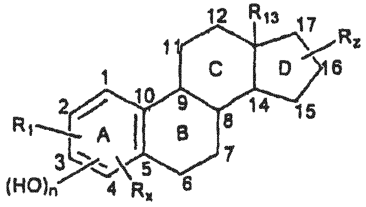 où: n est 1 ou 2; R₁ est un t-butyle; Rₓ est un hydrogène ou un alkyle; R₁₃ est un hydrogène ou un alkyle; et R_{z} est un hydrogène, un hydroxy, un alkyle ou un oxo.

2. Composé à utiliser selon la revendication 1, ayant la formule: 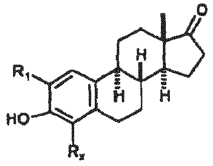 ou 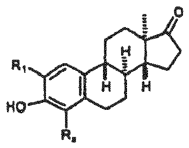 où R₁ et Rₓ sont tels que définis dans la revendication 1.

3. Composé à utiliser selon la revendication 1, ayant la formule: 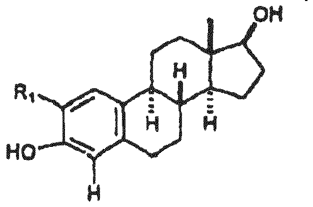 ou 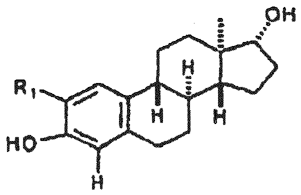 ou 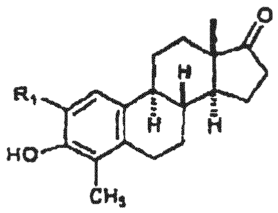 ou

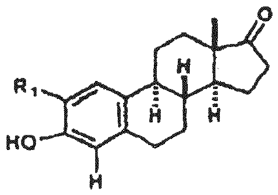

4. Composé à utiliser selon la revendication 1, ayant la formule: 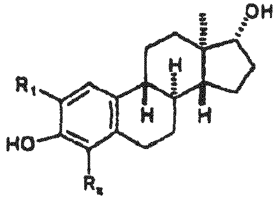 ou son énantiomère, où R₁ et Rₓ sont tels que définis dans la revendication 1.

5. Composé à utiliser selon l'une quelconque des revendications 1-4, où ledit traitement est le traitement d'un animal ayant ladite maladie.

6. Composé à utiliser selon l'une quelconque des revendications 1-4, où ledit traitement est le traitement d'un être humain ayant ladite maladie.

7. Composé à utiliser selon l'une quelconque des revendications 1-4, ledit composé étant administré à une population de cellules pour leur conférer une cytoprotection.

8. Composé à utiliser selon la revendication 7, où lesdites cellules sont des neurones.

9. Composé à utiliser selon la revendication 7, où lesdites cellules se trouvent dans le tissu ophtalmique.

10. Composé à utiliser selon l'une quelconque des revendications 1-9 en combinaison avec un support, excipient ou diluant pharmaceutiquement acceptable.

11. Composé selon l'une quelconque des revendications 1-10, à utiliser dans le traitement d'une maladie cytodégénérative choisie parmi la maladie d'Alzheimer, la maladie de Parkinson, la chorée de Huntington, la sclérose en plaques, la sclérose latérale amyotrophique, l'accident vasculaire cérébral, l'ischémie, les maladies ophtalmologiques, l'hémorragie vasculaire cérébrale, les démences, les lésions cérébrales induites par une drogue, et d'autres maladies systémiques ou aiguës **caractérisées par** une mort cellulaire nécrotique ou apoptotique.

12. Composé selon la revendication 11 à utiliser dans le traitement de maladies ophtalmologiques choisies parmi la dégénérescence maculaire, la dégénérescence du cristallin ou de la rétine, et la formation de cataractes ou d'un glaucome.

13. Composé ayant la formule: 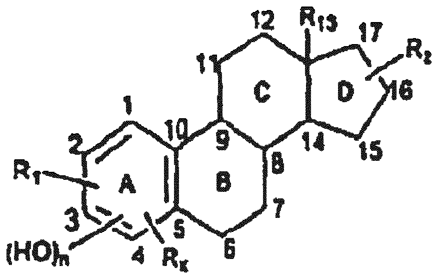 où: n est 1 ou 2; R₁ est un t-butyle; Rₓ est un hydrogène ou un alkyle; R₁₃ est un hydrogène ou un alkyle; et R_{z} est un hydrogène, un hydroxy, un alkyle ou un oxo, à condition que le composé n'ait pas l'une des formules suivantes:

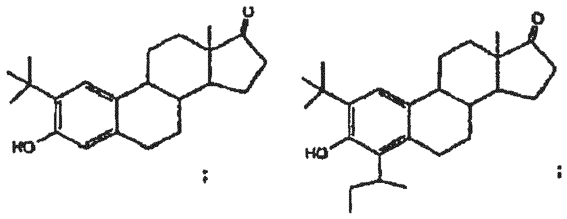

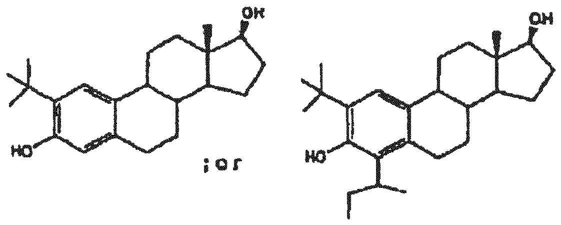

14. Composé selon la revendication 13, ledit composé ayant la formule: 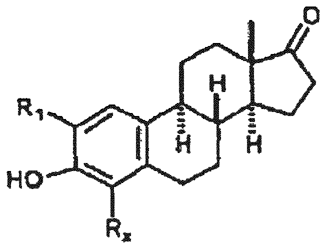 ou 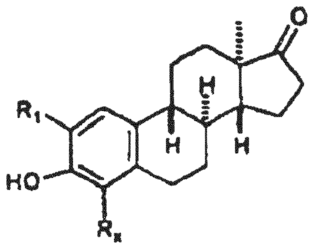 où R₁ est un t-butyle, et Rₓ est un groupe méthyle, éthyle, propyle, isopropyle, tert-butyle, pentyle ou hexyle.

15. Composé selon la revendication 13, le composé ayant la formule 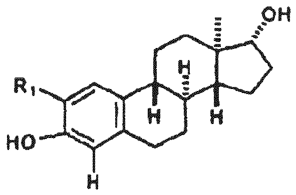 ou

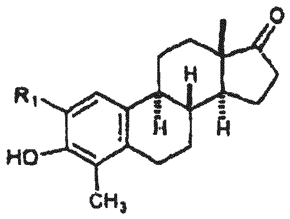

16. Composé selon la revendication 13, le composé ayant la formule: 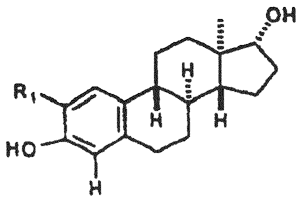 ou son énantiomère, où R₁ et Rₓ sont tels que définis dans la revendication 13.

17. Composé selon la revendication 13, où Rₓ est un alkyle, et est choisi parmi les groupes méthyle, éthyle, propyle, isopropyle, méthylpropyle, butyle, tert-butyle, pentyle ou hexyle.

18. Composé selon la revendication 13, où R₁₃ est un alkyle et est choisi parmi les groupes méthyle, éthyle, propyle, isopropyle, méthylpropyle, butyle, tert-butyle, pentyle ou hexyle.

19. Composé selon la revendication 13, où R_{z} est un alkyle et est choisi parmi les groupes méthyle, éthyle, propyle, isopropyle, méthylpropyle, butyle, tert-butyle, pentyle ou hexyle.

20. Composé selon la revendication 11, à utiliser dans le traitement de la maladie de Parkinson.
